(19) Europäisches Patentamt
European Patent Office
Office européen des brevets

(11) Numéro de publication: **0 275 224 B1**

(12) # FASCICULE DE BREVET EUROPEEN

(45) Date de publication de fascicule du brevet: **21.07.93**

(51) Int. Cl.[5]: **C07F 9/10**, C07H 17/065, A61K 31/70, A61K 31/685, A61K 7/48, C07D 311/62

(21) Numéro de dépôt: **88400052.2**

(22) Date de dépôt: **11.01.88**

(54) **Complexes phospholipidiques d'extraits de Vitis vinifera, leur procédé de préparation et compositions pharmaceutiques et cosmétiques les contenant.**

(30) Priorité: **14.01.87 IT 1908487**

(43) Date de publication de la demande: **20.07.88 Bulletin 88/29**

(45) Mention de la délivrance du brevet: **21.07.93 Bulletin 93/29**

(84) Etats contractants désignés: **AT BE CH DE ES FR GB GR IT LI LU NL SE**

(56) Documents cités:
**EP-A- 0 209 038**
**EP-A- 0 275 005**
**FR-A- 2 073 251**

**CHEMICAL ABSTRACTS, vol. 98, no. 13, 28 mars 1983, page 493, résumé no. 105933h, Columbus, Ohio, US; B.J.F. HUDSON et al.: "Polyhydroxy flavonoid antioxidants for edible oils. Phospholipids as synergists", & FOOD CHEM. 1983, 10(2), 111-20**

(73) Titulaire: **INDENA S.p.A.**
**Via Ripamonti, 99**
**I-20141 Milano(IT)**

Titulaire: **ELF SANOFI**
**32-34, rue Marbeuf**
**F-75008 Paris(FR)**

(72) Inventeur: **Bombardelli, Ezio**
**22 Via Valdi Sole**
**Milan(IT)**
Inventeur: **Sabadie, Michel**
**Rue de la Fontaine Saint Germain**
**27500 Bernay(FR)**

(74) Mandataire: **Le Guen, Gérard et al**
**CABINET LAVOIX 2, place d'Estienne d'Orves**
**F-75441 Paris Cédex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 275 224 B1

Rank Xerox (UK) Business Services
(3.10/3.6/3.3.1)

## Description

La présente invention concerne des complexes de phospholipides et d'extraits de *Vitis vinifera* , leur procédé de préparation et des compositions pharmaceutiques et cosmétiques les contenant.

Les extraits de *Vitis vinifera* qui peuvent être obtenus à partir des graines de la plante comme décrit dans les brevets GB-A-1541469 et FR-A-2092743, sont constitués d'un mélange de polyphénols dont, généralement, la (-) épicatéchine, les proanthocyanidines $B_1$ et $B_2$, la (+) catéchine et leurs dérivés de polymérisation, connus sous le nom d'oligomères procyanidoliques (dénomination commune française) et flavanoliques.

Les extraits de *Vitis Vinifera* sont actuellement utilisés en thérapeutique humaine principalement dans le traitement des maladies hépatiques, vasculaires ou en ophtalmologie.

Le mécanisme d'action pharmacologique de ces dérivés flavoniques, et spécialement des flavonoïdes catéchiques semble lié principalement à leur activité antioxydante et de pièges de radicaux libres qui se manifeste par leur action sur les processus de peroxydation lipidique, leur effet stabilisant des membranes et leur action sur la chaîne prostaglandinique, ainsi que par leur interaction avec les systèmes enzymatiques humoraux et tissulaires.

FR-A-2073251 décrit des complexes de flavonoïdes, mais avec des polyacides alcools.

CA-98 : 105 933 h décrit l'amélioration des propriétés antioxydantes de certains polyhydroxyflavonoïdes, en les mettant en présence de phospholipides, dans le saindoux à 100°-140°C.

EP-A-209 038 décrit des complexes de flavanolignanes et de phospholipides.

On a maintenant trouvé que les dérivés flavoniques présents dans les extraits de *Vitis Vinifera* forment avec des phospholipides naturels ou synthétiques des complexes aux propriétés pharmacologiques avantageuses et un premier objet de l'invention est constitué des complexes formés par réaction avec l'extrait des *Vitis vinifera* de phospholipides, d'origine végétale, animale ou synthétique, qui répondent à la formule générale :

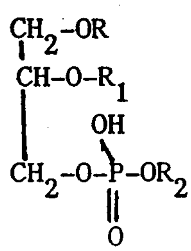

dans laquelle R et $R_1$ identiques ou différents représentent les groupes acyles des acides gras naturels tels que les acides palmitique, stéarique, oléique linoléique et linolénique tandis que $OR_2$ dérive d'un aminoalcool aliphatique de formule $R_2OH$, tel que $R_2$ représente $CH_2$-$CH_2$-$N^+(CH_3)_3$ ; $CH_2$-$CH_2$-$NH_2$ ; ou

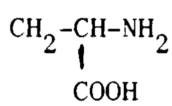

Les phospholipides préférés sont la lécithine de soja ou d'oeuf, les phospholipides de cervelle et de peau de boeuf ou de porc et les phosphatidylcholines, phosphatidylserines et phosphatidyléthanolamines, dont les groupes esters dérivent des acides gras précédemment cités.

Dans les complexes selon l'invention, le rapport du nombre de molécules de phospholipide à celui de flavonoïde et en général compris entre 0,5 et 3 et de préférence de 1.

Les complexes sont préparés par action des phospholipides sur les extraits flavoniques dans un solvant inerte ; on fait, en général, réagir de 1 à 3 parties en poids de phospholipide par partie d'extrait ; les complexes solubles sont isolés par évaporation du solvant sous vide, par lyophilisation ou après précipitation par addition d'un non solvant dans le milieu de préparation.

Les flavonoïdes sont en général insolubles dans les solvants apolaires et aprotiques comme les solvants chlorés $CHCl_3$ et $CH_2Cl_2$, tandis que les complexes le sont du fait de leur caractère lipophile ; par contre, les complexes sont décomposés dans les solvants à forte constante diélectrique comme le diméthylsulfoxyde et les alcools.

La formation de complexe entre les molécules de phospholipides et celles des flavonoïdes a été confirmée tout d'abord par l'étude des spectres RMN du proton du carbone 13 et du phosphore 31 de complexes de produits purs préparés à partir de certains des flavonoïdes présents dans l'extrait de *Vitis vinifera* et notamment avec la (+) catéchine, la (-) épicatechine et la proanthocyanidine $B_1$ sur lesquels on a fait réagir de la phosphatidylcholine de soja ou la distearoylphosphatidylcholine. Dans le spectre RMN$^1$-H du complexe, on n'observe plus les raies des protons de la molécule du flavonoïde contrairement à celles des protons de la partie lipidique et le massif des protons des méthyles du groupe $N(CH_3)_3$ de la choline est élargi par rapport au massif du produit pur. Les temps de relaxation des noyaux principalement concernés par la formation du complexe sont diminués jusqu'à faire disparaître sur le spectre RMN $^{13}C$ tous les signaux du flavonoïde et des groupes choline et glycéryle du phospholipide. Enfin, la raie du phosphore est élargie et déplacée sur le spectre du $^{31}P$.

Les médicaments comprenant ce complexe sont un autre objet de l'invention. En effet, les complexes de l'invention présentent, comme les oligomères flavanoliques de départ, une activité protectrice vasculaire ; notamment ils diminuent la perméabilité et la fragilité capillaire, comme le montrent les résultats obtenus dans l'essai de la perméabilité capillaire à l'histamine chez le rat, dont la méthodologie a été décrite par K. UDAKA et Col. dans : Proc. Soc. Exp. Biol. Med. 133, p 1384 (1970) ; cette activité est supérieure à celle des oligomères de départ.

Les complexes ont par ailleurs, lorsqu'ils sont administrés par voie locale, une activité antioedémateuse et cicatrisante que ne présentent pas les oligomères flavanoliques à partir desquels ils sont préparés. Cette activité est mise en évidence par le test d'inhibition de l'oedème à l'huile de croton dont la méthodologie est décrite par Tubaro et Coll. dans Agents and Actions 17 3/4 p. 347 -349 (1985).

Les médicaments de l'invention peuvent être utilisés sous une forme convenant pour l'administration par voie orale telle que comprimé, gélule ou pilule, pour le traitement des insuffisances veinolymphatiques telles que jambes lourdes, crampes, douleurs, paresthésies, oedèmes, ou pour le traitement des troubles de la circulation rétinienne et/ou choroïdienne ; ils seront administrés en régle générale à raison de 5 mg à 250 mg par jour, selon la nature et l'importance des troubles à traiter.

Les médicaments pour l'administration locale, se présenteront sous forme de crème contenant de 0,1 à 10 % en poids de principe actif qui pourra être appliquée une ou plusieurs fois par jour, sur la peau saine pour le traitement des jambes lourdes, des crampes et oedèmes notamment des membres inférieurs, ou sur les plaies pour favoriser leur cicatrisation ou encore en cas d'éruption cutanée pour diminuer l'oedème.

Les compositions cosmétiques comprenant des complexes de phospholidipides et des flavonoïdes des extraits de *Vitis vinifera* sont un autre objet de l'invention. Les complexes du fait de leurs propriétés antioxydantes, de capteurs de radicaux libres, de protection des lipides cutanés diminuent les atteintes des agents agressifs pour la peau et ralentissent son vieillissement.

Ils sont introduits avantageusement dans les produits de protection solaire, à utiliser avant ou après l'exposition au soleil, dans les produits de protection journalière, du type crème de jour ou base de maquillage, ou encore dans les produits de maquillage tels que les fonds de teints, les fards à joues ou à paupières, et les rouges et brillants à lèvres.

En outre, du fait de leur activité protectrice des microcapillaires cutanés, ils sont avantageusement introduits dans les produits anti-couperose.

En général, les complexes selon l'invention sont introduits dans les compositions cosmétiques à raison de 0,01 % à 1 % en poids.

Dans ce qui suit, on décrit des exemples de mise en oeuvre de l'invention ainsi que la préparation des complexes de (+) catéchine, de (-) épicatechine et de proanthyocianidine B, utilisés dans l'étude de résonnance magnétique nucléaire.

a) complexe de (+) catéchine et de distearoylphosphatidylcholine :
une suspension de 2,9 g de (+) catéchine et 8 g de distearoylphosphatidylcholine dans 50 ml de chlorure de méthylène est maintenue à la température de reflux du solvant jusqu'à solubilisation totale.

Après concentration, le résidu est introduit dans 150 ml de n-hexane où le complexe précipite. On obtient ainsi 10,5 g de produit blanc, de point de fusion 159-160 ° C et dont l'absorption UV à 280 nm dans $CH_3OH$ est $E_{1\%}$ = 39,4. Sur le spectre RMN-$^1$H, les raies des protons aromatiques entre 7 et 5,5 et celle des protons aliphatiques entre 2,3 et 4,8 et entre 3,9 et 4,6 (H sur les carbones porteurs de 0) et vers 3,4 (H sur les N(CH3)3) ont disparu.

b) complexe de la (-) épicatechine et de la phosphatidylcholine de soja.

Une solution de 2,9 g de (-) épicatechine et 7,8 g de phosphatidylcholine de soja à 95%, commercialisée sous la marque LIPOID S 100, dans 30 ml de dioxane est lyophilisée (?) pour donner 10,6 g d'un produit ayant un point de fusion de 170-172 ° C et $E_{1\%}$ = 40,7 à 280 nm ($CH_3OH$). Sur le spectre RMN$^1$-H dans $CHCl_3$, on observe des signaux non résolus entre 6,7-6,9 ; 5,1-5,3 ; 3,8-4,4 ; 2,6-3,1 ppm et ceux des protons oléfiniques à 5,2 ppm.

c) complexe de proanthocyanidine $B_1$, et de phosphatidylcholine de soja

Une solution de 5,8 g de proanthocyanidine $B_1$ et de 7,8 g de phosphatidylcholine de soja à 95 % , dans 100 ml d'un mélange de chlorure de méthylène et de méthanol 9/1 (V/V) est amenée à sec et le résidu redissous dans le chlorure de méthylène. La solution est alors concentrée jusqu'à 30 ml et versée dans 150 ml de n-hexane ; le précipité apparu, est isolé et séché sous vide à 60 ° C.

On obtient ainsi 13,1 g d'un complexe de point de fusion 128-129 ° C et $E_{1\%}$ = 75 à 280 nm dans $CH_3OH$

Sur le spectre RMN-$^1$H, dans $CDCl_3$, on observe de larges bandes non résolues entre 6,7-6,9 ; 5,1-5,3 et les raies des protons oléfiniques à 5,2 ppm, ceux des méthylènes entre 1,5 et 1,1 et des méthyles à 0,9 ppm.

**EXEMPLE 1** :Complexes d'un extrait purifié d'oligomères procyanidoliques de *Vitis vinifera* avec la phosphatidylcholine de soja.

L'extrait est préparé comme décrit dans FR-A-2092743

10 g de cet extrait et 15 g de phosphatidylcholine de soja à 95 % sont dissous dans 250 ml de chlorure de méthylène contenant 5 % en volume de méthanol. Le solvant est évaporé jusqu'à l'obtention d'un résidu pateux qui est dissous dans 300 ml de chlorure de méthylène. La solution est ensuite concentrée sous pression ordinaire, jusqu'à 50 ml et versée dans 300 ml de n-hexane.

Le précipité apparu est isolé et séché 24 heures sous vide à 60 ° C. On obtient 23 g d'un complexe, de point de fusion 105 ° C soluble dans les solvants apolaires. Sur le spectre RMN-$^1$H, on observe un massif entre 6,7 et 7 ppm, la raie des protons oléfiniques à 5,2 ppm, celle du méthylène entre 1,5 et 1,1 ppm et celle des méthyles à 0,9 ppm.

Dans le tableau I sont indiqués les résultats du test de la perméabilité capillaire à l'histamine :

**TABLEAU I**

| PRODUIT | NOMBRE D'ANIMAUX | DOSE | % D'INHIBITION |
|---|---|---|---|
| (témoin) | 8 | | |
| Oligomères procyanidoliques | 8 | 50 | - 16,81 |
| | 8 | 100 | - 22,05 |
| | 8 | 200 | - 22,44 |
| Complexe | 8 | 50 | - 23,51 |
| | 8 | 100 | - 26,96 |
| | 8 | 200 | - 30,69 |

Dans le tableau II sont reportés les résultats du test de l'oedème à l'huile de croton ; les produits ont été déposés sur la peau.

**TABLEAU II**

| PRODUIT | DOSE mg | NOMBRE ANIMAUX | OEDEME mg | inhibition % |
|---|---|---|---|---|
| (témoin) | - | 8 | 5,3 ± 0,5 | |
| oligomères | 300 | 8 | 4,7 ± 0,9 | 16 |
| complexes | 750 | 8 | 2,6 ± 0,4 | 54 |

**Exemple 2** : Complexe des oligomères procyanidoliques et de la distearoylphosphatidylcholine.

On introduit 3,2 g d'oligomères et 7,9 g de L-alpha-distearoylphosphatidylcholine à 99 % dans 50 ml d'un mélange de chlorure de méthylène et de méthanol 95/5 (V/V) ; après solubilisation la solution est concentrée et traitée comme décrit à l'exemple 1. On obtient ainsi 10,5 g de complexe de point de fusion 128,5° C.

Spectre RMN - $^1H$ ($CKCl_3$ :

Disparition des raies entre 6,4-7 ppm et de celles entre 8,7-9,2 ppm ; atténuation des signaux des méthylènes entre 1,5 et 1,1 ppm et élarissement du massif des $N-CH_3$ à 3,3 ppm.

**Exemple 3** : complexes d'un extrait de *Vitis Vinifera* avec un mélange naturel de phospholipides de soja.

On introduit dans 200 ml de dioxane, 10 g d'un extrait de *Vitis Vinifera* enrichi en oligomères procyanidoliques et 20 g d'un mélange naturel de phospholipides de soja à 97 % qui contient 30 % de phosphatidylcholine, 20 % de phosphatidyléthanolamine, 6 % de phosphatidylinositol et d'autres phospholipides.

La solution de dioxane est filtrée puis lyophilisée. On obtient ainsi 33,5 g de poudre beige claire, assez soluble dans les solvants chlorés et dont le spectre RMN-$^1H$ montre qu'il s'agit d'un complexe.

**Exemple 4** : complexe d'un extrait de *Vitis Vinifera* avec des phospholipides de soja hydrogénés.

10 g d'extrait et 15 g de lécithine de soja hydrogénée, commercialisée par NATTERMAN sous la marque Phospholipar-R100-H sont introduits dans 50 ml d'acétone, et celle-ci est portée au reflux.

La solution est alors concentrée sous vide et on introduit 150 ml d'heptane sur le résidu pâteux.

Le précipité formé est isolé par filtration.

Ce complexe est beige clair.

**Exemple 5** : Comprimés gastrorésistants d'un complexe de phosphatidylcholine de soja avec un extrait de *Vitis Vinifera*.

Des comprimés de 450 mg sont préparés de façon classique avec :

| | |
|---|---|
| Complexe de l'exemple 1 | 250 mg |
| Cellulose microcristalline | 118 mg |
| Silice précipitée | 3 mg |
| Stearate de Mg | 4 mg |
| Polymère anionique de l'acide méthacrylique et ses esters | 12 mg |
| Talc | 8 mg |
| Carbonate Mg | 8 mg |
| Amidon de maïs | 5 mg |
| Gomme arabique | 159 mg |

**Exemple 6** : Crème pharmaceutique.

On prépare de façon classique 100 g d'une crème pour application locale avec :

| | |
|---|---|
| Complexe de l'exemple 1 | 2 g |
| Alcool cétylique | 15 g |
| Myristate d'isopropyle | 5 g |
| Polymère carboxyvylique | 1 g |
| Laurylsarcosinate de sodium 3 | 3 g |
| Polysorbate 60 | 3 g |
| p-hydroxybenzoates | 0,2 g |
| Parfum | 0,2 g |
| Eau déminéralisée | q.s.p. |

**Exemple 7** : Emulsion solaire protectrice.

On prépare de façon classique 100 g d'émulsion avec :

| | |
|---|---|
| Huile végétale | 1,0 g |
| Huile minérale | 1,8 g |
| Alcools de lanoline | 0,2 g |
| Gel de bentone de caprate et de caprylate de propylène glycol | 5,0 g |
| Alcool cétylique | 1,7 g |
| Palmitate d'isopropyle | 4,0 g |
| Filtre UVB | 2,0 g |
| Filtre UVA | 2,0 g |
| Alcool laurique polyoxyéthylèné | 2,5 g |
| Stéarate de sorbitan | 2,5 g |
| Stéarate de sorbitan polyoxyéthylèné | 1,0 g |
| EDTA tétrasodique | 0,1 g |
| Carbopol | 0,35 g |
| Conservateurs dans butylèneglycol | 5,0 g |

| | |
|---|---|
| Parfum | 0,3 g |
| Complexe de l'exemple 1 | 0,2 g |
| Eau déminéralisée | 70,35 g |

**Exemple 8** : Gel solaire protecteur

| | |
|---|---|
| cérésine | 3,0 g |
| cire de riz | 3,0 g |
| huile végétale | 2,0 g |
| palmitate d'éthyl 2) hexyle | 44,7 g |
| gel de bentone de caprate et caprylate de propyléné glycol | 40,5 g |
| beurre de karité | 2,5 g |
| filtre UVA et filtre UVB | 2,0 g |
| complexe de l'exemple 1 | 0,2 g |
| colorant | 0,02 g |
| silice | 2 g |
| parfum | 0,08 g |

**Exemple 9** : Crème de jour protectrice

| | |
|---|---|
| stéarine | 1,75 g |
| monostéarate de propylèneglycol | 2,7 g |
| lanolate d'isopropyle | 3,5 g |
| gel de bentone de caprate et caprylate de propylène glycol | 6,0 g |
| palmitate d'isopropyle | 6,5 g |
| huile de silicone | 3,0 g |

| | |
|---|---|
| stéarate de sorbitan | 1,8 g |
| stéarate de sorbitan polyoxyéthylèné | 1,5 g |
| alcool cétylique | 0,6 g |
| filtre UVA et filtre UVB | 2,0 g |
| eau déminéralisée | 64,55 g |
| EDTA tétrasodique | 0,1 g |
| silicate d'Al | 0,8 g |
| carboxyméthylcellulose | 0,15 g |
| propylèneglycol | 4,0 g |
| conservateurs | 0,5 g |
| complexes de l'exemple 1 | 0,2 g |
| parfum | 0,35 g |

**Revendications**

**Revendications pour les Etats contractants suivants : AT, BE, CH, DE, FR, GB, IT, LI, LU, NL, SE**

1. Complexes de flavonoïdes extraits de *Vitis vinifera* et de phospholipides d'origine végétale, animale ou synthétique.

2. Complexes selon la revendication 1 caractérisés en ce que les phospholipides répondent à la formule :

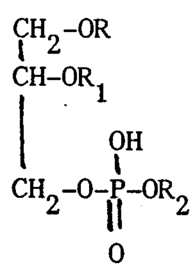

dans laquelle R et $R_1$ ,identiques ou différents, représentent les groupes acyles des acides gras et $OR_2$ représente un groupe dérivant d'un amino alcool aliphatique.

3. Complexes selon l'une des revendications 1 et 2 caractérisés en ce que les acides gras sont choisis parmi les acides palmitique, stéarique, oléique, linoléique, et linolénique.

4. Complexes selon l'une des revendications précédentes caractérisées en ce que les phospholipides sont choisis parmi la lécithine de soja ou d'oeuf, les phospholipides de cervelle ou de peau de bovins ou de porcins.

5. Complexes selon l'une des revendications précédentes caractérisés en ce que $R_2$ représente $(CH_2)_2$-N-$(CH_3)_3$ ; $(CH_2)_2NH_2$ou

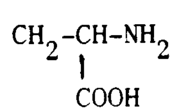

**6.** Complexes selon l'une quelconque des revendications 1 à 5, caractérisés en ce qu'ils sont obtenus par réaction de 1 à 3 parties en poids de phospholipide par partie d'extrait.

**7.** Procédé de préparation des complexes selon l'une des revendications précédentes caractérisé en ce que l'on fait réagir les phospholipides sur les flavonoïdes en solution dans un solvant et que l'on isole les complexes par évaporation du solvant, par précipitation par addition d'un non-solvant ou par lyophilisation.

**8.** Compositions pharmaceutiques contenant à titre de principe actif au moins un complexe selon l'une des revendications 1 à 6.

**9.** Compositions cosmétiques pour application sur la peau ou les muqueuses contenant au moins un complexe selon l'une des revendications 1 à 6.

**Revendications pour l'Etat contractant suivant : ES**

**1.** Procédé de préparation de complexes de flavonoïdes extraits de Vitis vinifera et de phospholipides d'origine végétale, animale ou synthétique, caractérisé en ce que l'on fait réagir les phospholipides sur les flavonoïdes en solution dans un solvant et que l'on isole les complexes par évaporation du solvant, par précipitation par addition d'un non-solvant ou par lyophilisation.

**2.** Procédé selon la revendication 1, caractérisé en que que les phospholipides répondent à la formule:

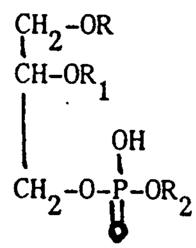

dans laquelle R et $R_1$, identiques ou différents, représentent les groupes acyles des acides gras et $OR_2$ représente un groupe dérivant d'un amino alcool aliphatique.

**3.** Procédé selon l'une des revendications 1 et 2, caractérisé en ce que les acides gras sont choisis parmi les acides palmitique, stéarique, oléique, linoléique, et linolénique.

**4.** Procédé selon l'une des revendications précédentes caractérisé en ce que les phospholipides sont choisis parmi la lécithine de soja ou d'oeuf, les phospholipides de cervelle ou de peau de bovins ou de porcins.

**5.** Procédé selon l'une des revendications précédentes caractérisé en ce que $R_2$ représente $(CH_2)_2$-N-$(CH_3)3$; $(CH_2)_2NH_2$ ou

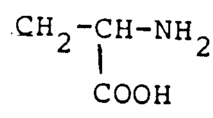

**6.** Procédé selon l'une quelconque des revendications 1 à 5, caractérisé en ce que l'un fait réagir de 1 à 3 parties en poids de phospholipide par partie d'extrait.

**7.** Procédé de préparation de compositions pharmaceutiques contenant à titre de principe actif au moins un complexe tel que défini dans l'une des revendications 1 à 6, consistant à mettre le principe actif sous une forme pharmaceutiquement acceptable.

**8.** Procédé de préparation de compositions cosmétiques pour application sur la peau ou les muqueuses contenant au moins un complexe tel que défini dans les revendications 1 à 6, consistant à mettre le complexe sous une forme cosmétique appropriée.

**9.** Compositions cosmétiques pour application sur la peau et les muqueuses contenant au moins un complexe tel que défini dans l'une des revendications 1 à 6.

**Revendications pour l'Etat contractant suivant : GR**

**1.** Complexes de flavonoïdes extraits de *Vitis vinifera* et de phospholipides d'origine végétale, animale ou synthétique.

**2.** Complexes selon la revendication 1 caractérisés en ce que les phospholipides répondent à la formule :

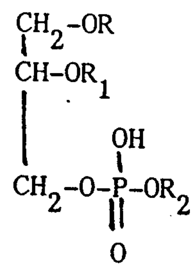

dans laquelle R et $R_1$ ,identiques ou différents, représentent les groupes acyles des acides gras et $OR_2$ représente un groupe dérivant d'un amino alcool aliphatique.

**3.** Complexes selon l'une des revendications 1 et 2 caractérisés en ce que les acides gras sont choisis parmi les acides palmitique, stéarique, oléique, linoléique, et linolénique.

**4.** Complexes selon l'une des revendications précédentes caractérisées en ce que les phospholipides sont choisis parmi la lécithine de soja ou d'oeuf, les phospholipides de cervelle ou de peau de bovins ou de porcins.

**5.** Complexes selon l'une des revendications précédentes caractérisés en ce que $R_2$ représente $(CH_2)_2$-N-$(CH_3)_3$; $(CH_2)_2NH_2$ ou

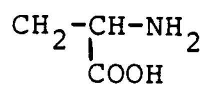

**6.** Complexes selon l'une quelconque des revendications 1 à 5, caractérisés en ce qu'ils sont obtenus par réaction de 1 à 3 parties en poids de phospholipide par partie d'extrait.

**7.** Procédé de préparation des complexes selon l'une des revendications précédentes caractérisé en ce que l'on fait réagir les phospholipides sur les flavonoïdes en solution dans un solvant et que l'on isole les complexes par évaporation du solvant, par précipitation par addition d'un non-solvant ou par lyophilisation.

**8.** Procédé de préparation de compositions pharmaceutiques contenant à titre de principe actif au moins un complexe selon l'une des revendications 1 à 6, consistant à mettre le principe actif sous une forme pharmaceutiquement acceptable.

**9.** Compositions cosmétiques pour application sur la peau ou les puqueuses contenant au moins un complexe selon l'une des revendications 1 à 6.

## Claims

**Claims for the following Contracting States : AT, BE, CH, DE, FR, GB, IT, LI, LU, NL, SE**

1. Complexes of flavonoids extracted from Vitis vinifera and from phospholipids of vegetable, animal or synthetic origin.

2. Complexes according to claim 1, characterized in that the complexes correspond to the formula:

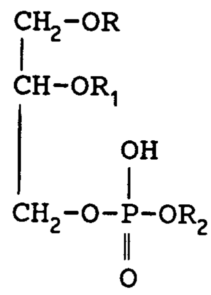

wherein R and $R_1$, which may be identical or different, denote the acyl groups of fatty acids and $OR_2$ denotes a group derived from an aliphatic amino alcohol.

3. Complexes according to one of claims 1 and 2, characterised in that the fatty acids are selected from the palmitic, stearic, oleic, linoleic and linolenic acids.

4. Complexes according to one of the preceding claims, characterized in that the phospholipids are selected from soya or egg lecithin, phospholipids of bovine or porcine brains or skin.

5. Complexes according to one of the preceding claims, characterized in that $R_2$ denotes $(CH_2)_2$-$N(CH_3)_3$; $(CH_2)_2NH_2$ or

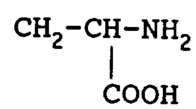

6. Complexes according to any one of claims 1 to 5, characterized in that they are obtained by reacting 1 to 3 parts by weight of the phospholipid with one part of extract.

7. Process for preparing the complexes according to one of the preceding claims, characterised in that the phospholipids are reacted with the flavonoids in solution in a solvent and the complexes are isolated by evaporation of the solvent, by precipitation by adding a non-solvent, or by lyophilisation.

8. Pharmaceutical compositions containing as active principle at least one complex according to one of claims 1 to 6.

9. Cosmetic compositions for application to the skin or mucosa, containing at least one complex according to one of claims 1 to 6.

**Claims for the following Contracting State : ES**

1. Process for preparing complexes of flavonoids extracted from Vitis vinifera and from phospholipids of vegetable, animal or synthetic origin, characterized in that the phospholipids are reacted with the flavonoids in solution in a solvent and the complexes are isolated by evaporation of the solvent, by precipitation by adding a non-solvent or by lyophilisation.

**2.** Process according to claim 1, characterized in that the phospholipids correspond to the formula:

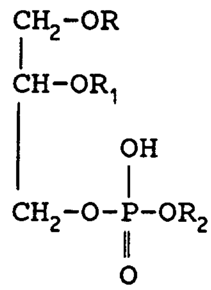

wherein R and $R_1$, which may be identical or different, denote the acyl groups of fatty acids and $OR_2$ denotes a group derived from an aliphatic amino alcohol.

**3.** Process according to one of claims 1 and 2, characterized in that the fatty acids are selected from the palmitic, stearic, oleic, linoleic and linolenic acids.

**4.** Process according to one of the preceding claims, characterized in that the phospholipids are selected from soya or egg lecithin, phospholipids of bovine or porcine brains or skin.

**5.** Process according to one of the preceding claims, characterized in that $R_2$ denotes $(CH_2)_2\text{-}N(CH_3)_3$; $(CH_2)_2NH_2$characterized in that $R_2$ denotes $(CH_2)_2\text{-}N(CH_3)_3$; $(CH_2)_2NH_2$ or

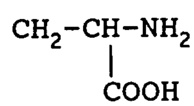

**6.** Process according to any one of claims 1 to 5, characterised in that 1 to 3 parts by weight of phospholipid are reacted per part of extract.

**7.** Process for preparing pharmaceutical compositions containing as active principle at least one complex as defined in one of claims 1 to 6, comprising putting the active principle in a pharmaceutically acceptable form.

**8.** Process for preparing cosmetic compositions for application to the skin or mucosa, containing at least one complex as defined in claims 1 to 6, comprising putting the complex in a suitable cosmetic form.

**9.** Cosmetic compositions for application to the skin and mucosa, containing at least one complex as defined in one of claims 1 to 6.

**Claims for the following Contracting State : GR**

**1.** Complexes of flavonoids extracted from Vitis vinifera and from phospholipids of vegetable, animal or synthetic origin.

**2.** Complexes according to claim 1, characterized in that the complexes correspond to the formula:

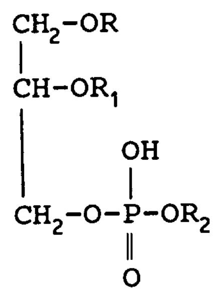

wherein R and $R_1$, which may be identical or different, denote the acyl groups of fatty acids and $OR_2$ denotes a group derived from an aliphatic amino alcohol.

**3.** Complexes according to one of claims 1 and 2, characterized in that the fatty acids are selected from the palmitic, stearic, oleic, linoleic and linolenic acids.

**4.** Complexes according to one of the preceding claims, characterized in that the phospholipids are selected from soya or egg lecithin, phospholipids of bovine or porcine brains or skin.

**5.** Complexes according to one of the preceding claims, characterized in that $R_2$ denotes $(CH_2)_2\text{-}N(CH_3)_3$; $(CH_2)_2NH_2$ or

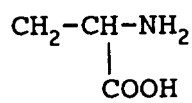

**6.** Complexes according to any one of claims 1 to 5, characterized in that they are obtained by reacting 1 to 3 parts by weight of the phospholipid with one part of extract.

**7.** Process for preparing the complexes according to one of the preceding claims, characterized in that the phospholipids are reacted with the flavonoids in solution in a solvent and the complexes are isolated by evaporation of the solvent, by precipitation by adding a non-solvent, or by lyophilisation.

**8.** Process for preparing pharmaceutical compositions containing as active principle at least one complex according to one of claims 1 to 6, comprising putting the active principle into a pharmaceutically acceptable form.

**9.** Cosmetic compositions for application to the skin or mucosa containing at least one complex according to one of claims 1 to 6.

**Patentansprüche**
**Patentansprüche für folgende Vertragsstaaten : AT, BE, CH, DE, FR, GB, IT, LI, LU, NL, SE**

**1.** Komplexe von aus Vitis vinifera extrahierten Flavonoiden und Phospholipiden pflanzlichen, tierischen oder synthetischen Ursprungs.

**2.** Komplexe nach Anspruch 1, dadurch gekennzeichnet, daß die Phospholipide folgende Formel aufweisen:

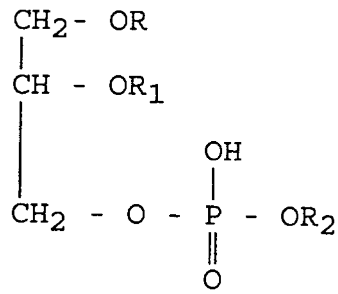

in der R und $R_1$ gleich oder verschieden sind und Acylgruppen von Fettsäuren bedeuten und $OR_2$ eine von einem aliphatischen Aminoalkohol abgeleitete Gruppe bedeutet.

**3.** Komplexe nach einem der Ansprüche 1 oder 2, dadurch gekennzeichnet, daß die Fettsäuren unter Palmitinsäure, Stearinsäure, Ölsäure, Linolsäure und Linolensäure ausgewählt sind.

**4.** Komplexe nach einem der vorstehenden Ansprüche, dadurch gekennzeichnet, daß die Phospholipide unter Lecithin aus Soja oder Ei und Phospholipiden aus dem Hirn oder der Haut von Rindern oder Schweinen ausgewählt sind.

**5.** Komplexe nach einem der vorstehenden Ansprüche, dadurch gekennzeichnet, daß $R_2$ die Bedeutung $(CH_2)_2 - N(CH_3)_3$, $(CH_2)_2NH_2$ oder

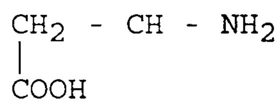

hat.

**6.** Komplexe nach einem der Ansprüche 1 bis 5, dadurch gekennzeichnet, daß sie durch Umsetzung von 1 bis 3 Gewichtsteilen Phospholipid pro 1 Teil des Extraktes erhalten worden sind.

**7.** Verfahren zur Herstellung der Komplexe nach einem der vorstehenden Ansprüche, dadurch gekennzeichnet, daß man die Phospholipide mit den Flavonoiden in Lösung in einem Lösungsmittel umsetzt und die Komplexe durch Verdampfen des Lösungsmittels, durch Ausfällen durch Zugabe eines Nicht-Lösungsmittels oder durch Lyophilisieren isoliert.

**8.** Pharmazeutische Zusammensetzungen, die als Wirkstoff mindestens einen Komplex nach einem der Ansprüche 1 bis 6 enthalten.

**9.** Kosmetische Zusammensetzungen zur Anwendung auf der Haut oder den Schleimhäuten, die mindestens einen Komplex nach einem der Ansprüche 1 bis 6 enthalten.

**Patentansprüche für folgenden Vertragsstaat : ES**

**1.** Verfahren zur Herstellung von Komplexen von aus Vitis vinifera extrahierten Flavonoiden und Phospholipiden pflanzlichen, tierischen oder synthetischen Ursprungs, dadurch gekennzeichnet, daß man die Phospholipide mit den Flavonoiden in Lösung in einem Lösungsmittel umsetzt und die Komplexe durch Verdampfen des Lösungsmittels, durch Ausfällen durch Zugabe eines Nicht-Lösungsmittels oder durch Lyophilisieren isoliert.

**2.** Verfahern nach Anspruch 1, dadurch gekennzeichnet, daß die Phospholipide folgende Formel aufweisen:

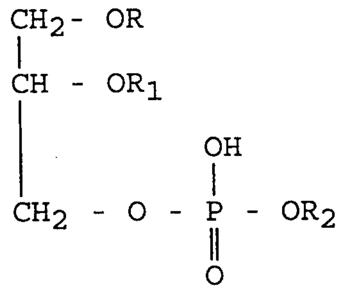

in der R und $R_1$ gleich oder verschieden sind und Acylgruppen von Fettsäuren bedeuten und $OR_2$ eine von einem aliphatischen Aminoalkohol abgeleitete Gruppe bedeutet.

**3.** Verfahren nach einem der Ansprüche 1 oder 2, dadurch gekennzeichnet, daß die Fettsäuren unter Palmitinsäure, Stearinsäure, Ölsäure, Linolsäure und Linolensäure ausgewählt sind.

**4.** Verfahren nach einem der vorstehenden Ansprüche, dadurch gekennzeichnet, daß die Phospholipide unter Lecithin aus Soja oder Ei und Phospholipiden aus dem Hirn oder der Haut von Rindern oder Schweinen ausgewählt sind.

**5.** Verfahren nach einem der vorstehenden Ansprüche, dadurch gekennzeichnet, daß $R_2$ die Bedeutung $(CH_2)_2 - N(CH_3)_3$, $(CH_2)_2NH_2$ oder

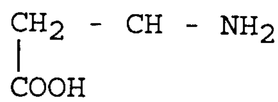

hat.

**6.** Verfahren nach einem der Ansprüche 1 bis 5, dadurch gekennzeichnet, daß man 1 bis 3 Gewichtsteile Phospholipid pro 1 Teil des Extraktes umsetzt.

**7.** Verfahren zur Herstellung von pharmazeutischen Zusammensetzungen, die als Wirkstoff mindestens einen Komplex gemäß der Definition in den Ansprüchen 1 bis 6 enthalten, wobei das Verfahren darin besteht, daß man den Wirkstoff in eine pharmazeutisch akzeptable Form bringt.

**8.** Verfahren zur Herstellung von kosmetischen Zusammensetzungen zur Anwendung auf der Haut oder den Schleimhäuten, die mindestens einen Komplex gemäß der Definition in den Ansprüchen 1 bis 6 enthalten, wobei das Verfahren darin besteht, daß man den Komplex in eine kosmetisch geeignete Form bringt.

**9.** Kosmetische Zusammensetzungen zur Anwendung auf der Haut und den Schleimhäuten, die mindestens einen Komplex gemäß der Definition in den Ansprüchen 1 bis 6 enthalten.

**Patentansprüche für folgenden Vertragsstaat : GR**

**1.** Komplexe von aus Vitis vinifera extrahierten Flavonoiden und Phospholipiden pflanzlichen, tierischen oder synthetischen Ursprungs.

**2.** Komplexe nach Anspruch 1, dadurch gekennzeichnet, daß die Phospholipide folgende Formel aufweisen:

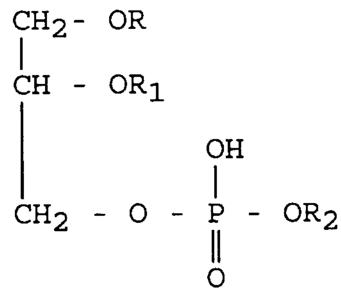

in der R und $R_1$ gleich oder verschieden sind und Acylgruppen von Fettsäuren bedeuten und $OR_2$ eine von einem aliphatischen Aminoalkohol abgeleitete Gruppe bedeutet.

**3.** Komplexe nach einem der Ansprüche 1 oder 2, dadurch gekennzeichnet, daß die Fettsäuren unter Palmitinsäure, Stearinsäure, Ölsäure, Linolsäure und Linolensäure ausgewählt sind.

**4.** Komplexe nach einem der vorstehenden Ansprüche, dadurch gekennzeichnet, daß die Phospholipide unter Lecithin aus Soja oder Ei und Phospholipiden aus dem Hirn oder der Haut von Rindern oder Schweinen ausgewählt sind.

**5.** Komplexe nach einem der vorstehenden Ansprüche, dadurch gekennzeichnet, daß $R_2$ die Bedeutung $(CH_2)_2 - N(CH_3)_3$, $(CH_2)_2NH_2$ oder

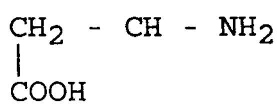

hat.

**6.** Komplexe nach einem der Ansprüche 1 bis 5, dadurch gekennzeichnet, daß sie durch Umsetzung von 1 bis 3 Gewichtsteilen Phospholipid pro 1 Teil des Extraktes erhalten worden sind.

**7.** Verfahren zur Herstellung der Komplexe nach einem der vorstehenden Ansprüche, dadurch gekennzeichnet, daß man die Phospholipide mit den Flavonoiden in Lösung in einem Lösungsmittel umsetzt und die Komplexe durch Verdampfen des Lösungsmittels, durch Ausfällen durch Zugabe eines Nicht-Lösungsmittels oder durch Lyophilisieren isoliert.

**8.** Verfahren zur Herstellung von pharmazeutischen Zusammensetzungen, die als Wirkstoff mindestens einen Komplex nach einem der Ansprüche 1 bis 6 enthalten, wobei das Verfahren darin besteht, daß man den Wirkstoff in eine pharmazeutisch akzeptable Form bringt.

**9.** Kosmetische Zusammensetzungen zur Anwendung auf der Haut oder den Schleimhäuten, die mindestens einen Komplex nach einem der Ansprüche 1 bis 6 enthalten.